Europäisches Patentamt

European Patent Office

Office européen des brevets

(19)

(11) Numéro de publication : **0 102 369**
**B1**

(12) **FASCICULE DE BREVET EUROPÉEN**

(45) Date de publication du fascicule du brevet :
12.11.86

(21) Numéro de dépôt : 83900825.7

(22) Date de dépôt : 23.02.83

(86) Numéro de dépôt international :
PCT/BE 83/00005

(87) Numéro de publication internationale :
WO/8302898 (01.09.83 Gazette 83/20)

(51) Int. Cl.⁴ : **A 61 L 15/07, C 08 L 67/04**

(54) COMPOSITION DE MATIERES POUR ELEMENTS DE CONTENTION ET PLAQUES A BASE DE CETTE COMPOSITION ET UTILISATION DESDITES PLAQUES.

(30) Priorité : 24.02.82 LU 83972

(43) Date de publication de la demande :
14.03.84 Bulletin 84/11

(45) Mention de la délivrance du brevet :
12.11.86 Bulletin 86/46

(84) Etats contractants désignés :
AT BE CH DE FR GB LI NL SE

(56) Documents cités :
FR-A- 2 042 813
FR-A- 2 099 473
FR-A- 2 208 640
FR-A- 2 376 170
Le dossier contient des informations techniques présentées postérieurement au dépôt de la demande et ne figurant pas dans le présent fascicule.

(73) Titulaire : HEXCEL CORPORATION
650 California Street Suite 1400
San Francisco, CA 94108 (US)

(72) Inventeur : LIEGEOIS, Jean-Marie
176, Moulin de Wadeleux
B-4654 Charneux-Herve (BE)

(74) Mandataire : van Malderen, Michel et al
p.a. FREYLINGER & ASSOCIES 85/042 Boulevard de
la Sauvenière
B-4000 Liège (BE)

EP 0 102 369 B1

## Description

La présente invention concerne des compositions de matières permettant notamment la réalisation de matériaux de contentions ou d'empreintes pour des prothèses externes ou d'autres éléments de fixation, et elle s'étend à des plaques réalisées à partir de tels matériaux et à leurs applications.

On sait que les contentions destinées à immobiliser les membres fracturés ou à faciliter la rééducation de malformations ont été réalisées dans le passé à base de plâtre de Paris ou à base d'éléments de cuir éventuellement renforcés par des structures métalliques.

Les contentions à base de plâtre de même que les contentions faites à l'aide de cuir doivent cependant être préparées en atelier et occasionnent un délai de plusieurs jours avant leur utilisation. En outre, elles sont souvent inconfortables et en tout cas représentent un poids important qui doit être supporté par le patient. Les contentions à base de cuir sont particulièrement sujettes à vieillissement qui provoque la perte progressive de la rigidité nécessaire.

Certains polymères synthétiques ou naturels ont été également utilisés plus récemment pour la réalisation de tels matériaux. Dans ce cas cependant les contentions sont préfabriquées en différentes tailles et pour les différentes parties du corps humain qu'il faut généralement soutenir. Il est rare que ces formes préfabriquées, non remodelables, s'adaptent parfaitement au contour des membres à soutenir. Pour cette raison, une couche de mousse est placée entre la peau et la contention de manière à compenser le jeu qui varie d'un endroit à un autre. Cette façon de faire n'apporte pas tout le confort souhaité et empêche notamment de pouvoir appliquer une force de soutien suffisante aux endroits précis où celle-ci serait souhaitable.

On utilise également des plaques à base de polycaprolactone ou à base de trans-1-4-polyisoprène (gutta-percha) que l'on peut former à chaud au moment de l'application.

Ces matériaux polymériques apparus plus récemment ne répondent cependant pas non plus aux exigences qui sont légitimement attendues d'un tel appareillage. En effet, bien que ces dits matériaux offrent certains avantages par rapport au plâtre, au cuir et aux contentions préformées, comme par exemple la possibilité d'être façonnés au moment de l'application, réduisant ainsi le délai de mise en application, ils présentent cependant un certain nombre d'inconvénients. Il faut, en effet, tenir compte du fait que ce façonnage fait intervenir un ramollissement à chaud des plaques réalisées en ces matériaux en vue de leur donner la forme désirée pour s'adapter de manière adéquate aux éléments du corps humain qui doivent être soutenus. Or, on observe qu'il est généralement difficile de donner une forme rigoureusement correcte épousant parfaitement les parties du corps humain comme la musculature, les pommettes, etc. Le trans-1-4-polyisoprène présente notamment l'inconvénient de conserver une élasticité excessive à chaud qui empêche le fluage nécessaire au formage. L'opérateur est ainsi obligé de maintenir sous tension les déformations qu'il souhaite conférer à la contention pendant le refroidissement du matériau jusqu'à son durcissement complet. En outre, celui-ci est aussi le siège de tensions internes qui peuvent occasionner le retrait et un retour à la forme initiale dans le cas où, soit par accident, soit volontairement dans le but de remodeler les contentions, celles-ci seraient réchauffées. Un autre inconvénient majeur des contentions faites de trans-1-4-polyisoprène est la nécessité d'utiliser un adhésif le plus souvent sous forme d'une dissolution de polymère en vue d'assurer le collage d'une partie de la contention sur une autre ou bien de joindre deux éléments l'un à l'autre.

D'autre part, les contentions réalisées à partir de polycaprolactone offrent l'inconvénient, contrairement au trans-1-4-polyisoprène, de s'allonger de manière excessive sous de faibles efforts de sorte que le fluage à chaud au moment du formage est excessif et empêche généralement de réaliser la forme précise que l'on veut obtenir.

On connaît également des matériaux à base de polycaprolactone réticulé qui présentent le même désavantage que le trans-1-4-polyisoprène à savoir une inaptitude à se déformer de manière stable.

Par le document FR-A-2 042 813, on connaît de nouveaux mélanges de polymère cristallins contenant des polyalcènes cristallins tels que le polyéthylène ou le polypropylène, en association intime avec des polymères cristallins d'ester cyclique. Ce document est néanmoins relatif à une composition cristalline à caractéristiques particulières, qui ne convient pas pour des matériaux de contention, empreintes pour prothèses externes ou autres éléments de fixation qui ne peuvent être constitué d'une matière cristalline.

Par le document FR-A-2 376 170, on connaît une composition synthétique destinée à des moulages orthopédiques. Néanmoins, ce document fait appel à un support, comme une bande de gaze de coton. Par ailleurs, le composé polymérisé se déforme facilement et risque de « couler » à la manière d'un liquide visqueux, lors de sa mise en œuvre. C'est d'ailleurs une des raisons pour lesquelles il est utilisé en combinaison avec un support adéquat.

La présente invention a pour objet une composition de matière qui convient tout particulièrement pour réaliser des contentions du type décrit ci-dessus et qui permet également d'autres usages telle que la réalisation d'empreintes pour des prothèses externes, etc.

L'invention s'étend aux plaques réalisées à partir de tels matériaux qui sont modelables facilement à chaud par le praticien et qui présentent les propriétés physiques souhaitées pour permettre un formage par élongation et une déformation correcte en fonction de la forme souhaitée. L'invention s'étend également aux applications de ces compositions et de ces plaques pour la réalisation de contentions et d'empreintes pour prothèses externes.

Le produit obtenu selon l'invention présente une élongation convenable à chaud qui permet de façonner convenablement la contention ou l'empreinte désirée sans aucune élasticité gênante.

De plus, le produit est suffisamment adhésif sur lui-même au moment du formage de manière à permettre une adhérence sur lui-même et/ou sur d'autres éléments de support sans recours à des colles ou similaires.

L'invention porte de manière spécifique sur une composition de matières caractérisée en ce qu'elle est constituée à raison de 90 à 60 parts, de préférence d'environ 70 parts en poids de polymère d'ester cyclique de poids moléculaire de 20 000 à 300 000 et de 10 à 40 parts, de préférence 30 parts en poids de copolymère éthylène-acétate de vinyle (EVA) d'un indice de viscosité au fondu de 0,5 à 12 (selon la norme ASTM D-1238) et présentant une teneur en acétate de vinyle d'au moins 10 et d'au plus 60 % en poids, suffisante pour dispenser de substrat.

Suivant un mode d'exécution particulièrement préféré de l'invention, le polymère d'ester cyclique choisi sera du poly-ε-caprolactone.

Selon l'invention, le matériau d'éléments de contention peut comporter pour 100 parties du mélange de polymères précité, 2 à 20 parties, de préférence 10 parties de fibres courtes de longueur moyenne de 0,2 à 4 mm et d'un denier de 0,5 à 8 (0,06 à 0,89 Tex).

Avantageusement, on peut également ajouter, avec ou sans présence de fibres courtes précitées, pour 100 parties en poids du mélange de polymères précité, 5 à 40 parties, de préférence 13 parties d'une charge minérale. Cette charge minérale peut avoir été traitée en surface ou non et sera de préférence de forme sphérulitique ou aciculaire.

On peut encore éventuellement ajouter, en présence ou non desdites fibres courtes et de ladite charge minérale, pour 100 parties en poids du mélange de polymère, 1 à 5 parties en poids d'un pigment tel que l'oxyde de titane sur des plaques préparées à l'aide des compositions précitées, ces plaques étant obtenues par exemple par extrusion à travers une filière plate.

L'invention, ainsi qu'il a été indiqué précédemment, s'étend à l'application desdites plaques pour la réalisation de contentions ou d'empreintes pour prothèses externes. Ces plaques peuvent être soit pleines, soit perforées de trous, soit encore aérées de quelque nature que ce soit. En pratique on provoque d'abord un ramollissement desdites plaques dans un bain maintenu à une température de 60 à 80 °C et ensuite on provoque le formage soit directement sur le membre à soutenir, soit d'abord sur un mannequin, par exemple. Ce ramollissement peut être effectué dans tout autre moyen tel que dans une étuve, ou dans un four à rayonnement. Les fibres textiles utilisées dans le procédé de l'invention sont choisies en fonction des propriétés souhaitées. Il convient de noter que les fibres textiles devront de préférence présenter de bonnes caractéristiques de « mouillabilité » par les résines ; à cet effet, on choisira de préférence des fibres courtes non ramifiées, le choix des fibres particulières utilisées étant réalisé de manière à obtenir une bonne dispersion dans le mélange de polymère. A titre d'exemple de fibres entrant dans les compositions de l'invention, on peut citer les fibres de nylon, de polyester, des mélanges de nylon et de viscose, ainsi que les fibres de coton et les fibres vinyliques.

Les charges minérales que l'on peut utiliser sont par exemple des charges minérales de carbonate de calcium notamment du carbonate de calcium précipité.

Le choix du pigment est dicté essentiellement par des considérations de coloration à conférer au produit en fonction des exigences particulières.

Il convient de noter que lorsqu'il est question dans le présent mémoire descriptif d'un polymère d'ester cyclique ou plus particulièrement d'un poly-ε-caprolactone, il faut entendre un produit qui est essentiellement composé de chaînons de cet ester cyclique ou de caprolactones, sans que ceux-ci excluent la présence de quantités faibles d'un autre monomère dans la structure du polymère ou la présence de groupes terminaux différents de ceux constituant le polymère pur.

De la même manière, le copolymère éthylène-acétate de vinyle peut comporter en petites portions d'autres co-monomères pour autant que le caractère général d'un copolymère éthylène-acétate de vinyle soit préservé.

Afin d'illustrer la mise en pratique de l'invention, on se référera utilement aux exemples qui suivent qui sont décrits uniquement à titre d'illustration de l'invention, sans aucun caractère limitatif.

Exemple 1

Dans un mélangeur à pales tournant à vitesse lente d'une capacité de 0,5 litre, on dispose 105 grammes de granulés de polycaprolactone de masse moléculaire 45 000 et 45 grammes de granulés d'un copolymère d'éthylène et acétate de vinyle de viscosité au fondu de 3 et contenant 28 % d'acétate de vinyle. Ce mélange est ensuite transféré sur un malaxeur à cylindres chauffés à 120 °C où les résines sont fondues. On obtient un mélange homogène qui est repris dans une presse à plateaux où l'on forme une plaque de 3 mm d'épaisseur. Après refroidissement la plaque est sortie de la presse, cette plaque est rigide et d'une grande solidité. En immergeant cette plaque dans de l'eau préchauffée à 75 °C, on obtient une pièce souple facilement déformable qui peut suivre les contours des doigts d'une main. Après durcissement on retrouve une empreinte qui peut servir de contention pour maintenir les doigts de la main dans la position où ils étaient au moment du formage. Cette composition adhère bien sur elle-même à chaud.

Exemple 2

En procédant comme dans l'exemple 1, on a ajouté à ces deux résines 18 g de fibres courtes de nylon de 6 deniers et de 1 mm de longueur ainsi que 27 g de carbonate de calcium traité en surface et 7,5 g de dioxyde de titane. On a obtenu après malaxage et moulage, une plaque blanche d'une grande rigidité à froid et qui se déforme de manière aisément contrôlée lorsqu'on l'a plongée dans un bain d'eau à 70 °C. Cette composition adhère bien sur elle-même à chaud.

Exemple 3

En suivant la procédure exposée dans l'exemple 1, on a ajouté au mélange de résine 18 g de fibres courtes de nylon de 2 deniers et d'une longueur de 0,5 mm ainsi que 18 g de carbonate de calcium sphérulitique traité par de l'acide stéarique et 7,5 g de dioxyde de titane.

On obtient après malaxage et refroidissement dans la presse, une plaque blanche rigide et résistante qui peut facilement être déformée à chaud et épouser les formes les plus complexes pour réaliser une contention correcte.

Exemple 4

On prépare dans un mélangeur d'une capacité de 200 litres, le mélange à froid de 30 kg de résine polycaprolactone de poids moléculaire 45 000 et 10 kg d'un copolymère d'alkylène-acétate de vinyle de viscosité au fondu de 3 et 28 % de teneur en acétate de vinyle, on y ajoute 13,2 kg de carbonate de calcium spérulitique traité en surface et 1,68 kg de dioxyde de titane. Le mélange est alors traité dans une extrudeuse équipée d'une filière plate d'une largeur de 60 cm et d'une ouverture de 3 mm. La température dans l'extrudeuse est de 120 °C. On recueille après refroidissement au sortir de la filière, une bande continue dans laquelle on découpe les plaques pour réaliser des éléments de contention précis par traitement à 70 °C, température à laquelle le produit adhère bien sur lui-même et sur d'autres objets divers utilisables en association avec la contention.

Exemple 5

Utilisant la même technique que dans l'exemple 4 on traite après mélange, 4 050 g de polycaprolactone, 1 350 g de copolymère éthylène-acétate de vinyle, 1 080 g de carbonate de calcium aciculaire et 540 g de fibres courtes de coton. On obtient après extrusion, à travers une filière plate, des plaques résistantes et rigides qui se déforment correctement à chaud et conviennent pour former des éléments de contention corrects et précis.

## Revendications

1. Composition pour matériaux de contentions, empreintes pour prothèses externes ou autres éléments de fixation, caractérisée en ce qu'elle est constituée à raison de 90 à 60 parts en poids de polymère d'ester cyclique de poids moléculaire compris entre 20 000 et 300 000 et 10 à 40 parts en poids de copolymère éthylène-acétate de vinyle d'indice de viscosité au fondu de 0,5 à 12 et présentant une teneur en acétate de vinyle d'au moins 10 et d'au plus 60 % en poids, suffisante pour dispenser de substrat.

2. Composition suivant la revendication 1 caractérisée en ce que le polymère d'ester cyclique est essentiellement composé de poly-ε-caprolactones.

3. Composition selon l'une des revendications 1 ou 2 caractérisée en ce que la teneur en polymère d'ester cyclique ou poly-ε-caprolactone est de l'ordre d'environ 70 parts en poids, le complément jusqu'à 100 parts étant constitué par un copolymère éthylène-acétate de vinyle.

4. Composition selon l'une des revendications précédentes caractérisée en ce qu'elle comporte environ 30 parts en poids de copolymère éthylène-acétate de vinyle, le restant de la composition étant constitué essentiellement de polymère d'ester cyclique ou de poly-ε-caprolactone.

5. Matériaux d'éléments de contentions caractérisés en ce qu'ils contiennent pour 100 parties en poids de polymère, 2 à 20 parties en poids de fibres courtes de longueur moyenne de 0,2 à 4 mm et d'un denier de 0,5 à 8 (0,06 à 0,89 Tex).

6. Matériaux d'éléments de contentions selon la revendication 5 caractérisés en ce qu'ils comportent également pour 100 parties en poids de polymère, de 5 à 40 parties en poids d'une charge minérale.

7. Matériaux selon la revendication 6 caractérisés en ce que ladite charge minérale est traitée en surface.

8. Matériaux selon la revendication 6 caractérisés en ce que la charge minérale n'a pas été traitée en surface.

9. Matériaux selon l'une quelconque des revendications 6 à 8 caractérisés en ce que ladite charge minérale est de forme sphérulitique ou aciculaire.

10. Matériaux selon l'une quelconque des revendications 5 à 9 caractérisés en ce qu'ils comportent de 1 à 5 parties en poids d'un pigment pour 100 parties en poids de polymère.

11. Plaques de matières convenant notamment comme matériaux de contentions ou de matériaux pour empreintes destinées à des prothèses externes, caractérisées en ce qu'elles sont réalisées à l'aide de la composition selon l'une quelconque des revendications 1 à 10.

12. Plaques selon la revendication 11 réalisées par extrusion à l'aide d'une filière plate.

13. Plaques selon l'une des revendications 11 ou 12 caractérisées en ce qu'elles sont aérées.

14. Application des plaques selon la revendication 11 ou 12 à la réalisation d'éléments de contentions ou d'empreintes pour prothèses externes.

## Claims

1. Composition for retention materials, impressions for external prostheses or other fixing elements, characterized in that it consists of 90 to 60 parts by weight of a polymer of a cyclic ester of a molecular weight between 20,000 and 300,000, and 10 to 40 parts by weight of an ethylene-vinyl acetate copolymer with a melt viscosity index from 0.5 to 12 and having a vinyl acetate content from at least 10 to maximum 60 % by weight, sufficient to relieve from the use of a substrate.

2. Composition according to claim 1 characterized in that the polymer of a cyclic ester consists substantially of poly-ε-caprolactones.

3. Composition according to one of claims 1 or 2 characterized in that the content of a polymer of a cyclic ester or poly-ε-caprolactone is of the order of approximately 70 parts by weight, the remainder up to 100 parts consisting of an ethylene-vinyl acetate copolymer.

4. Composition according to one of the preceeding claims, characterized in that it comprises approximately 30 parts by weight of an ethylene-vinyl acetate copolymer, the remainder of the composition consisting substantially of a polymer of a cyclic ester or of poly-ε-caprolactone.

5. Materials for retention elements characterized in that it contains 2 to 20 parts by weight of short fibers of average lenght from 0.2 to 4 mm and of a denier from 0.5 to 8 (0.06 to 0.89 Tex), for 100 parts by weight of polymer.

6. Material for retention elements according to claim 5 characterized in that it also comprises from 5 to 40 parts by weight of a mineral filler for 100 parts by weight of polymer.

7. Material according to claim 6 characterized in that said mineral filler is surface-treated.

8. Material according to claim 6 characterized in that the mineral filler has not been surface-treated.

9. Material according to any one of claims 6 to 8 characterized in that said mineral filler is of spherulitic or acicular form.

10. Material according to any one of claims 5 to 9 characterized in that it comprises 1 to 5 parts by weight of a pigment for 100 parts by weight of polymer.

11. Plates of materials particularly appropriate as retention materials or materials for impressions intended for external prostheses, characterized in that they are produced with the aid of the composition according to any one of claims 1 to 10.

12. Plates according to claim 11 produced by extrusion with the aid of a flat die.

13. Plates according to one of claims 11 or 12 characterized in that they are aired ; .

14. Application of the plates according to claim 11 or 12 for the production of retention elements or impressions for external prostheses.

## Patentansprüche

1. Verbindung für Stützverbandmaterial, Außenprotesenmatrizen oder andere Stützelemente, dadurch gekennzeichnet, daß sie aus 90 bis 60 Gewichtsteilen eines Polymeres von zyklischen Estern mit einem Molekulargewicht zwischen 20 000 und 300 000 und aus 10 bis 40 Gewichtsteilen eines Mischpolymerisats von Ethylen und Vinylacetat mit einem Schmeltz-Viskositätsindex von 0,5 bis 12 und einem Gehalt an Vinylacetat von mindestens 10 und höchstens 60 Gewichtsprozent — genügend um sich von der Verwendung eines Substrats zu befreien — besteht.

2. Verbindung nach Anspruch 1, dadurch gekennzeichnet, daß das Polymer von zyklischen Estern im wesentlichen Poly-ε-Caprolacton enthält.

3. Verbindung nach einem der Ansprüche 1 oder 2, dadurch gekennzeichnet, daß der Gehalt an Polymer von zyklischen Estern oder an Poly-ε-Caprolacton um ungefähr 70 Gewichtsteilen liegt, wobei das Komplement zu 100 aus einem Mischpolymerisat von Ethylen und Vinylacetat besteht.

4. Verbindung nach einem der vorherigen Ansprüche, dadurch gekennzeichnet, daß sie ungefähr 30 Gewichtsteile Mischpolymerisat von Ethylen und Vinylacetat enthält, wobei der Rest der Verbindung im wesentlichen aus dem Polymer von zyklischen estern oder Poly-ε-Caprolacton besteht.

5. Stützverbandmaterial, dadurch gekennzeichnet, daß es für 100 Gewichtsteile Polymer, 2 bis 20 Gewichtsteile kurze Fasern mit einer mittleren Länge von 0,2 bis 4 mm und einem Denier von 0,5 bis 8 (0,06 bis 0,89 Tex) enthält.

6. Stützverbandmaterial nach Anspruch 5, dadurch gekennzeichnet, daß es ebenfalls, für 100 Gewichtsteile Polymer, 5 bis 40 Gewichtsteile eines anorganischen Füllmittels enthält.

7. Stützverbandmaterial nach Anspruch 6, dadurch gekennzeichnet, daß das genannte Füllmittel oberflächen-behandelt ist.

8. Stützverbandmaterial nach Anspruch 6, dadurch gekennzeichnet, daß das genannte Füllmittel nicht oberflächen-behandelt ist.

9. Stützverbandmaterial nach einem der Ansprüche 6 bis 8, dadurch gekennzeichnet, daß das genannte anorganischen Füllmittel sphärolitisch oder nadelförmig ist.

10. Stützverbandmaterial nach einem der Ansprüche 5 bis 9, dadurch gekennzeichnet, daß es 1 bis 5 Gewichtsteile eines Farbpigments, für 100 Gewichtsteile Polymer, enthält.

11. Platten aus einem Material für Stützverbände oder Außenprotesenmatrizen, dadurch gekennzeichnet, daß sie aus der Verbindung nach einem der Anpsrüche 1 bis 10 hergestellt sind.

12. Platten nach Anspruch 11 hergestellt durch

Strangpressen in einem flachen Extruderwerkzeug.

13. Platten nach einem des Ansprüche 11 oder 12, dadurch gekennzeichnet, daß sie mit Belüftungsöffnungen versehen sind.

14. Verwendung der Platten nach Anspruch 11 oder 12 zur Herstellung von Stützverbandmaterialien oder Außenprotesenmatrizen.